(19) Europäisches Patentamt
European Patent Office
Office européen des brevets

(11) Veröffentlichungsnummer : **0 381 623 B1**

(12) **EUROPÄISCHE PATENTSCHRIFT**

(45) Veröffentlichungstag der Patentschrift : **31.03.93 Patentblatt 93/13**

(51) Int. Cl.$^5$ : **C07C 323/22,** C07C 309/08, C07C 43/12, C07D 307/20

(21) Anmeldenummer : **90810057.1**

(22) Anmeldetag : **24.01.90**

(54) **Verfahren zur Herstellung substituierter Cyclopropancarbaldehyde.**

(30) Priorität : **02.02.89 CH 365/89**

(43) Veröffentlichungstag der Anmeldung : **08.08.90 Patentblatt 90/32**

(45) Bekanntmachung des Hinweises auf die Patenterteilung : **31.03.93 Patentblatt 93/13**

(84) Benannte Vertragsstaaten : **AT BE CH DE DK ES FR GB GR IT LI LU NL SE**

(56) Entgegenhaltungen :
**EP-A- 0 243 313**
**DE-A- 2 120 908**

(73) Patentinhaber : **CIBA-GEIGY AG**
**Klybeckstrasse 141**
**CH-4002 Basel (CH)**

(72) Erfinder : **Känel, Hans-Ruedi, Dr.**
**Mittelbrühlstrasse 32**
**CH-4416 Bubendorf (CH)**
Erfinder : **Dingwall, John Grey, Dr.**
**St. Pantaleonstrasse 16**
**CH-4412 Nuglar (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 381 623 B1

Jouve, 18, rue Saint-Denis, 75001 PARIS

## Beschreibung

Die Erfindung betrifft ein neues Verfahren zur Synthese 1-alkylthio- bzw. 1-benzylthiosubstituierter Cyclopropancarbaldehyde der Formel

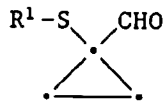

(I) ,

worin
$R^1$ $C_1$-$C_4$-Alkyl oder Benzyl bedeutet.

Die Cyclopropancarbaldehyde der Formel I sind wertvolle Zwischenverbindungen zur Synthese agrochemischer Wirkstoffe. So etwa zur Herstellung der aus der DE-A-21 20 908 bekannt gewordenen Cycloalkancarbaldoximcarbamate oder zur Synthese der in der EP-A-0 243 313 beschriebenen herbizid und pflanzenwuchsregulatorisch wirksamen 2-Acyl-cyclohexan-1,3-dione.

Diese Cyclcohexandione sind unter anderem in Position 5 durch einen 1-Alkylthio- bzw einen 1-Benzylthio-cycloprop-1-yl-Rest substituiert. Sie können durch eine Malonestersynthese ausgehend von 1-Alkylthio- bzw. 1-Benzylthiocyclopropancarboxaldehyden dargestellt werden. Dabei wird zunächst der Aldehyd mit Aceton zu dem entsprechenden 4-(1-Alkyl-(oder Benzyl-)thio)-cyclopropyl-but-3-en-2-on kondensiert und danach mit Malonester oder Cyanessigsäureester zum Cyclohexandion cyclisiert. Das so erhältliche Cyclohexandion wird danach in an sich bekannter Weise in der Stellung 2 funktionalisiert.

Aus der DE-A-2 120 908 ist ein Verfahren zur Herstellung von Verbindungen der Formel I, worin $R^1$ $C_1$-$C_4$-Alkyl bedeutet, bekannt geworden.

In diesem fünfstufigen Verfahren wird Chloracetonitril über Alkylthioacetonitril zu einem 1-Alkylthiocyclopropancarbonitril umgesetzt. Diese Verbindung wird dann verseift, zunächst in das Säurechlorid und anschliessend in ein Amid überführt und mittels $LiAlH_4$ zum Aldehyd reduziert.

Dieses Verfahren hat zahlreiche Nachteile. Neben der schlechten Gesamtausbeute des Verfahrens, sind auch für die einzelnen Reaktionsstufen aufwendige Verfahrensschritte erforderlich. Ausserdem ist das als Ausgangsverbindung benötigte Alkylthioacetonitril korrosiv und tränenreizend.

Demgegenüber betrifft die Erfindung ein Verfahren zur Herstellung von Cyclopropancarbaldehyden der Formel

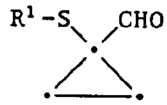

(I) ,

worin
$R^1$ $C_1$-$C_4$-Alkyl oder Benzyl bedeutet,
dadurch gekennzeichnet, dass man

a) ein Tetrahydrofuran der Formel

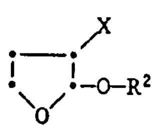

(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel zu einem 1,2,4-Trihalogenbutylether der Formel

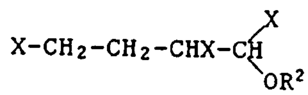

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
umsetzt und
b) anschliessend die Verbindung der Formel III mit einer wässrigen Bisulfitlösung zu einem Bisulfitaddukt der Formel

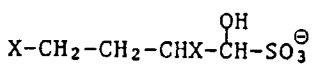 $Y^{\oplus}$ (IV),

worin
X Halogen und
Y ein Kationenäquivalent eines Alkali- oder Erdalkaliions bedeutet,
umsetzt und
c) anschliessend in Gegenwart einer Base mit einem Thiolat der Formel

$$R^1 - S^{\ominus}\ Z^{\oplus} \quad (V),$$

worin
$R^1$ $C_1$-$C_4$-Alkyl oder Benzyl und
Z ein Kationenäquivalent eines Alkali- oder Erdalkaliions bedeutet,
zu einem Cyclopropancarbaldehyd der Formel I cyclisiert.

Die Verfahrensschritte a), b) und c) sind neu. Die Erfindung betrifft sowohl die Kombination aller drei Verfahrensstufen, wie zuvor beschrieben, als auch die Kombination der Verfahrensstufen a) + b) und b) + c) und die Einzelstufen a), b) und c) allein.

Die 1,2,4-Trihalogenbutylether der Formel III und die Bisulfitaddukte der Formel IV sind ebenfalls neu. Sie bilden einen weiteren Gegenstand vorliegender Erfindung.

Die Erfindung betrifft somit die neuen 1,2,4-Trihalogenbutlyether der Formel

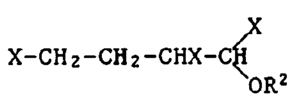

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
und die neuen Bisulfitaddukte der Formel

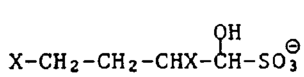 $Y^{\oplus}$ (IV),

worin
X Halogen und
Y ein Kationenäquivalent eines Alkali- oder Erdalkaliions bedeutet.

Die Tetrahydrofurane der Formel II sind wertvolle Ausgangsverbindungen für das erfindungsgemässe Verfahren. Einige dieser Verbindungen sind literaturbekannt. Die Verbindungen der Formel II können analog zu literaturbekannten Verfahren hergestellt werden. So bietet zum einen die Alkoholyse von 2,3-Dihalogentetrahydrofuranen [M. Holik et al.; (Chem. Zvesti; 25 (1971),9); V. Zezula und M. Kratochvil; Collect. Czech. Chem. Commun.; 35 (1970), 1745], [L.M. Bolotina et al.; Khim. Geterotsikl. Soedin; 4 (1968), 200] einen bequemen Zugang zu dieser Verbindungsklasse. Die Zn bzw. $ZnCl_2$-katalysierte Addition von Oxiranen an 2,3-Dichlortetrahydrofuran oder Mischungen aus 2,3-Dichlortetrahydrofuran mit Tetrahydrofuran stellt einen weiteren bequemen Zugang zu 2-[2- oder 4-chloralkylsubstituierten]-3-chlortetrahydrofuranen der Formel II dar [M. Kratochvil, Collect. Czech, Chem. Commun. 25 (1960), 1353].

Die Erfindung betrifft auch die neuen Tetrahydrofurane der Formel II

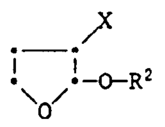

(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit der Massgabe, dass
wenn X Chlor bedeutet $R^2$ nicht für unsubstituiertes $C_1$-$C_6$-Alkyl, Cyclohexyl, in 2-Stellung durch Chlor substituiertes $C_4$-$C_{10}$-Alkyl oder 4-Chlorbutyl steht oder wenn X Brom oder Iod bedeutet,
$R^2$ nicht für $C_1$-$C_4$-Alkyl steht.

Die einzelnen erfindungsgemässen Verfahrensstufen sowie deren Kombinationen sind wie folgt definiert:

Als zweistufige Verfahren, das

Verfahren zur Herstellung von Cyclopropancarbaldehyden der Formel

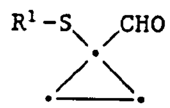

(I) ,

worin
$R^1$ $C_1$-$C_4$-Alkyl oder Benzyl bedeutet,
dadurch gekennzeichnet, dass man

b) einen 1,2,4-Trihalogenbutylether der Formel

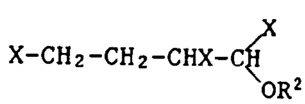

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einer wässrigen Bisulfitlösung zu einem Bisulfitaddukt der Formel

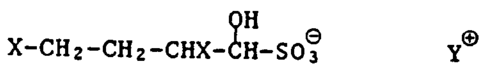

(IV),

worin
X Halogen und
Y ein Kationenäquivalent eines Alkali- oder Erdalkaliions bedeutet,
umsetzt
und
c) danach in Gegenwart einer Base mit einem Thiolat der Formel

$$R^1 - S^{\ominus} Z^{\oplus} \quad (V),$$

worin
$R^1$ $C_1$-$C_4$-Alkyl oder Benzyl und
Z ein Kationenäquivalent eines Alkali- oder Erdalkaliions bedeutet, zu einem Cyclopropancarbaldehyd der Formel I cyclisiert;
und das
Verfahren zur Herstellung eines Bisulfitaddukts der Formel

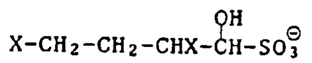 (IV),

worin
X Halogen und
Y ein Kationenäquivalent eines Alkali- oder Erdalkaliions bedeutet,
dadurch gekennzeichnet, dass man
a) ein Tetrahydrofuran der Formel

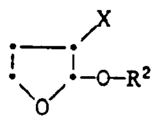 (II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel zu einem 1,2,4-Trihalogenbutylether der Formel

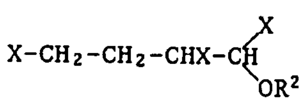 (III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet, umsetzt
und
b) anschliessend die Verbindung der Formel III mit einer wässrigen Bisulfitlösung zu dem Bisulfitaddukt der Formel IV umsetzt.
Als einstufige Verfahren, das
Verfahren zur Herstellung von 1,2,4-Trihalogenbutylether der Formel

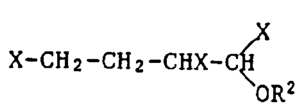 (III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
dadurch gekennzeichnet, dass man
a) ein Tetrahydrofuran der Formel

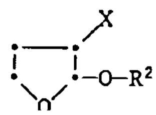
(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel umsetzt;

das Verfahren zur Herstellung eines Bisulfitaddukts der Formel

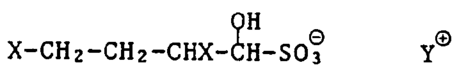
(IV),

worin
X Halogen und
Y ein Kationenäquivalent eines Alkali- oder Erdalkaliions bedeutet,
dadurch gekennzeichnet, dass man
b) einen 1,2,4-Trihalogenbutylether der Formel

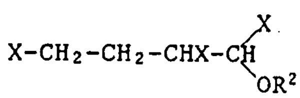
(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einer wässrigen Bisulfitlösung umsetzt;

und das Verfahren zur Herstellung von Cyclopropancarbaldehyden der Formel

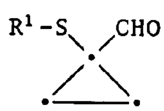
(I) ,

worin
$R^1$ $C_1$-$C_4$-Alkyl oder Benzyl bedeutet,
dadurch gekennzeichnet, dass man
c) ein Bisulfitaddukt der Formel

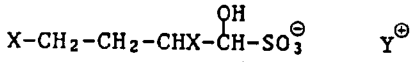
(IV),

worin
X Halogen und
Y ein Kationenäquivalent eines Alkali- oder Erdalkaliions bedeutet, in Gegenwart einer Base mit einem Thiolat der Formel

$$R^1 - S^{\ominus} Z^{\oplus} \quad (V),$$

worin
$R^1$ $C_1$-$C_4$-Alkyl oder Benzyl und
Z ein Kationenäquivalent eines Alkali- oder Erdalkaliions bedeutet,
zu einem Cyclopropanarbaldehyd der Formel I cyclisiert.

In den in dieser Beschreibung verwendeten Definitionen betreffen die verwendeten generischen Begriffe insbesondere die folgenden Einzelbedeutungen:

Alkyl ist Methyl, Ethyl, n-Propyl, iso-Propyl, n-Butyl, iso-Butyl, sec.-Butyl und tert.-Butyl, sowie die isomeren Pentyl-, Hexyl-, Heptyl-, Octyl-, Nonyl- und Decylradikale. Im Falle von $R^2$ als Alkyl ist $C_1$-$C_4$-Alkyl bevorzugt.

Halogen ist Fluor, Chlor, Brom und Iod, insbesondere Chlor und Brom.

Alkali- und Erdalkalimetallionen sind insbesondere Lithium, Natrium, Kalium sowie die zweiwertigen Ionen von Magnesium und Calcium. Im Falle der Bisulfitaddukte sind die Natrium- und Kaliumsalze bevorzugt, ganz besonders bevorzugt sind die Natriumsalze.

Halogenalkyl im Falle des Substituenten $R^2$ sind insbesondere 2-Chlorethyl, 2-Chlorpropyl, 2-Chlorbutyl und 4-Chlorbutyl.

Cycloalkyl ist Cyclopropyl, Cyclobutyl, Cyclopentyl und Cyclohexyl vorzugsweise Cyclopentyl und Cyclohexyl. Die Cycloalkylreste können ihrerseits durch Halogen, Alkyl oder Alkoxy substituiert sein. Bevorzugt sind die Cycloalkylreste unsubstituiert oder bis zu dreifach gleich oder verschieden durch Halogen, $C_1$-$C_4$-Alkyl oder $C_1$-$C_4$-Alkoxy substituiert.

$R^2$ als Phenyl oder Benzyl ist vorzugsweise unsubstituiert, kann aber ebenfalls bis zu dreifach gleich oder verschieden durch Halogen, $C_1$-$C_4$-Alkyl, $C_1$-$C_4$-Alkoxy, Nitro oder Cyano substituiert sein. $R^2$ als Alkyl ist vorzugsweise unsubstituiertes $C_1$-$C_4$-Alkyl.

Im Falle der zwei- bzw. des dreistufigen Verfahren (Kombination der Verfahrensschritte b) + c); a) + b); oder a) + b) + c)) können die Synthesen mit Vorteil als "Eintopfverfahren", also ohne Isolierung der jeweiligen Zwischenprodukte durchgeführt werden.

Für die einzelnen Verfahrensstufen haben sich folgende Massnahmen als vorteilhaft erwiesen:

Verfahrensstufe a)

Die Halogenierung des Tetrahydrofurans II wird mit Erfolg in Gegenwart von 0,OO5 bis 0,5 Mol eines Katalysators, gegebenenfalls mit 0,0001 bis 0,5 Mol eines Protonendonors, 1 bis 6 Mol des Halogenierungsmittels und 0 bis 200 ml eines reaktionsinerten Lösungsmittels je Mol Edukt durchgeführt. Geeignete Protonendonatoren sind beispielsweise Wasser, Alkohole, Säuren wie Salzsäure, Schwefelsäure, Carbonsäuren, Sulfonsäuren sowie Ammoniumionen (mit Ausnahme der quaternären). Bevorzugte Protonendonatoren sind Wasser, Alkohole und Salzsäure. In besonders vorteilhafter Weise wird die Verfahrensstufe a) ohne Lösungsmittel in Gegenwart von O,O2 bis 0,1 Mol eines Katalysators gegebenenfalls mit 0,001 bis 0,01 Mol Wasser und 1,10 bis 1,15 Mol Halogenierungsmittel durchgeführt.

Als Halogenierungsmittel besonders bevorzugt sind jene Halogenierungsmittel, deren Rückstände aufgrund ihrer leichten Flüchtigkeit aus dem Reaktionsgemisch leicht abgetrennt werden können, wie etwa Thionylchlorid oder Thionylbromid. Thionylchlorid ist in besonderem Masse geeignet.

Als Katalysatoren zu nennen sind vor allem aromatische Amine wie Pyridin, Lutidin, 4-Dimethylaminopyridin, Chinolin, Picolin, tertiäre Amine wie N,N-Dimethylaniline, 4-N',N'-Dimethylaminopyridine oder 1,4-Diazabicyclo[2.2.2]octan, aber auch Trialkylamine, wie Triethylamin oder Ethyldiisopropylamin oder Amide wie Dimethylformamid, Dimethylacetamid oder Hexamethylphosphortriamid.

Geeignete Lösungsmittel sind unter anderem aromatische Kohlenwasserstoffe, wie Toluol, Xylol oder Benzol; Ether, wie Tetrahydrofuran, Dioxan, Diethylether oder Diisopropylether; alicyclische oder aliphatische Kohlenwasserstoffe, wie Cyclohexan, Hexan, oder höhersiedende Alkane; chlorierte Kohlenwasserstoffe, wie Dichlormethan oder Trichlormethan.

Das Verfahren wird vorzugsweise bei Temperaturen von Raumtemperatur bis etwa 140°C, insbesondere im Temperaturbereich von 30 bis 100°C und besonders bevorzugt zwischen 80 und 85°C durchgeführt.

In einer besonders bevorzugten Ausführungsform wird das Verfahren in Gegenwart von O,O2 bis O,1 Mol Pyridin oder Triethylamin als Katalysator, 1,10 bis 1,15 Mol Thionylchlorid und ohne Lösungsmittel bei der Siedetemperatur des Reaktionsgemisches durchgeführt. Bei dieser vorteilhaften Ausführungsform des Verfahrensschrittes a) entsteht als Produkt ein 1,4-Dichlor-2-halogenbutylether der Formel

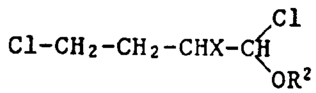 (IIIa)

worin X für Halogen und $R^2$ für gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl steht.

Verfahrensstufe b)

Die Hydrolyse des Ethers III zum Bisulfitaddukt IV wird mit Vorteil in einem wässrigen Zweiphasensystem unter kontrollierten pH-Bedingungen durchgeführt. Dabei ergeben sich im einzelnen, bei der Verwendung einer 40%igen Natriumbisulfitlösung, die folgenden bevorzugten und besonders bevorzugten Verhältnisse von Lösungsmitteln und Edukten:

Bevorzugt sind 1,0 bis 2,O Mol Natriumbisulfit (als 40%ige wässrige Lösung), 20 bis 200 ml Wasser und 20 bis 200 ml eines reaktionsinerten organischen Lösungsmittels je Mol Ether III. Besonders bevorzugt sind 1,0 bis 1,1 Mol Natriumbisulfit (als 40%ige wässrige Lösung), 40 bis 60 ml Wasser und 40 bis 60 ml organisches Lösungsmittel je Mol Ether III.

Die Reaktion wird mit Vorteil bei Temperaturen von O bis 100°C, vorzugsweise bei Temperaturen zwischen 30 und 80°C durchgeführt.

Dabei wird der pH-Wert des Reaktionsgemisches durch kontrollierte Zugabe einer Base im pH-Bereich von etwa 2 bis 7, vorzugsweise zwischen 3,0 und 3,5 gehalten. Als Base geeignet ist NaOH oder KOH (etwa als 30%ige wässrige Lösung).

Vorzugsweise wird für die Durchführung des Verfahrensschritts b) ein Ether der Formel IIIa verwendet.

Die für die Verfahrensstufe b) geeigneten Lösungsmittel entsprechen den in Stufe a) genannten. Zusätzlich geeignet sind Ester wie etwa Essigsäureethylester. Besonders bevorzugt ist Toluol.

Das mit der Verfahrensstufe b) erhältliche Bisulfitaddukt kann in einfacher Weise erhalten werden, indem man das Reaktionsgemisch zunächst leicht erwärmt und bei dieser erhöhten Temperatur von der organischen Phase abtrennt. Aus der wässrigen Phase kristallisiert das Produkt IV mit hoher Reinheit und kann entweder abgetrennt oder aber als wässrige Suspension direkt in Verfahrensstufe c) weiterverarbeitet werden.

Verfahrensstufe c)

Das Bisulfitaddukt IV wird in 400 bis 1000 ml, vorzugsweise 600 bis 800 ml, Wasser je Mol Edukt suspendiert oder aber die nach Verfahrensstufe b) erhältliche Suspension wird direkt verwendet. Dazu werden bei Temperaturen von -20 bis +40°C, vorzugsweise 0 bis 5°C 1,0 bis 1,5 Mol, vorzugsweise 1,0 bis 1,05 Mol, Thiolat V zugegeben. Das Reaktionsgemisch wird mit 1,02 bis 2,20 Mol, vorzugsweise mit 1,02 bis 2,0 Mol, Natronlauge auf einen basischen pH-Wert eingestellt. Aus dem Reaktionsgemisch kann das Produkt anschliessend extrahiert werden.

Für die Durchführung der Verfahrensstufe c) werden Bisulfitaddukte der Formel

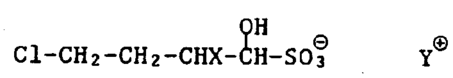 (IVa)

worin
X Halogen und
Y ein Kationenäquivalent eines Alkali- oder Erdalkaliions bedeutet,
besonders bevorzugt.

Als Thiolate sind die Natrium und Kaliumthiolate der Formel V bevorzugt.

Als Basen in den vorgenannten Reaktionsstufen b) oder c) können neben Natronlauge auch andere Alkali- und Erdalkalimetallhydroxide oder -carbonate eingesetzt werden.

Das erfindungsgemässe Verfahren zeichnet sich durch zahlreiche Vorteile gegenüber dem aus dem Stand der Technik Bekannten aus. Es geht von einfach zugänglichen Edukten und Reagenzien aus. Die Reaktionsführung ist unkompliziert (Temperatur, Lösungsmittel, pH-Kontrolle, Verwendung von Wasser als Reaktionsmedium, Abtrennung der Produkte, gute Entsorgung der Nebenprodukte und Rückstände etc.). Es kann mit oder ohne Isolierung der Zwischenstufen gearbeitet werden. Das Bisulfitaddukt IV ist lagerstabil. Ausserdem weist das Verfahren in jeder einzelnen Reaktionsstufe eine hohe Ausbeute und hohe Produktqualität auf.

Die nachstehenden Beispiele erläutern die Erfindung.

Herstellungsbeispiele

H. 1. Herstellung von (1,2,4-Trichlorbut-1-yl)-methylether

Zu 136,5 g (1,0 Mol) 3-Chlor-2-methoxytetrahydrofuran und 1,58 g (0,02 Mol) Pyridin werden bei 80-85°C innerhalb von 2 Stunden 130,9 g (1,1 Mol) Thionylchlorid zugetropft, wobei sofortige Gasentwicklung einsetzt. Es wird bis zur Beendigung der Gasentwicklung weitergerührt und danach auf Raumtemperatur abgekühlt.

Man isoliert 185,7 g (97 %) der Titelverbindung der Formel

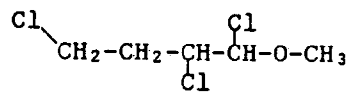

als hellbraune Flüssigkeit (Verb. No. 1.01).

Spektroskopische Daten für die Verbindung No. 1.01.

$^1$H-NMR (90 MHz, $CDCl_3$):

| ppm | Identifizierung |
|---|---|
| 1,9 - 2,9 | Multiplett 2H-C(3) |
| 3,4 - 4,0 | Multiplett 2H-C(4) |
| 3,63 | Singulett $H_3C$-O |
| 4,1 - 4,6 | Multiplett H-C(2) |
| 4,68 und 4,76 | 2 Doubletts, Verhältnis 1:2, J = 7 und J = 4 H-C(1) |

MS: 157(10), 155(16), 121(2), 119(7), 107(3), 105(10), 94(2), 92(5), 81(34), 79(100), 55(3), 53(5), 51(13); M = 191,5; (für beide Diastereomere)

Analog können die Verbindungen der Tabelle I (unter Verwendung von $SOCl_2$ bzw. $SOBr_2$ als Halogenierungsmittel) hergestellt werden.

Tabelle I

Verbindungen der Formel

$$X'-CH_2-CH_2-CHX''-\overset{\overset{\displaystyle X'''}{|}}{C}H-OR^2 \qquad (III)$$

| Verb. No. | X' | X'' | X''' | $R^2$ | Sdp./Druck | Ausbeute |
|---|---|---|---|---|---|---|
| 1.01 | Cl | Cl | Cl | $CH_3$ | +100°C/5,3 Pa | 97 % |
| 1.02 | Br | Cl | Br | $CH_3$ | | |
| 1.03 | Br | Br | Br | $CH_3$ | | |
| 1.04 | Cl | Br | Cl | $CH_3$ | +85°C/2,7 Pa | 96,8 % |
| 1.05 | Cl | Cl | Cl | $C_2H_5$ | | |
| 1.06 | Br | Cl | Br | $C_2H_5$ | | |
| 1.07 | Br | Br | Br | $C_2H_5$ | | |
| 1.08 | Cl | Br | Cl | $C_2H_5$ | | |

## H. 2. Herstellung der Bisulfitaddukte IV

### H. 2.1. Herstellung des Bisulfitaddukts des 2,4-Dichlorbutyraldehyds

Zu 310,0 g (1,0 Mol) einer 33,5%igen wässrigen Natriumhydrogensulfitlösung und 50 ml Toluol werden bei 40 - 45°C innerhalb einer Stunde 185,6 g (0,97 Mol) (1,2,4-Trichlorbut-1-yl)-methylether zugetropft. Gleichzeitig wird der pH-Wert mit 30%iger wässriger NaOH-Lösung bei pH 3,0 bis 3,5 gehalten. Anschliessend wird das Reaktionsgemisch auf 70 - 75° erwärmt und die Toluolphase abgetrennt und verworfen. Beim Abkühlen der wässrigen Phase auf 15°C kristallisiert das Produkt aus.

Man isoliert 162,1 g (66 %) der Titelverbindung der Formel

$$Cl-CH_2-CH_2-CHCl-\overset{\overset{\displaystyle OH}{|}}{C}H-SO_3^{\ominus} \qquad Na^{\oplus}$$

als farbloses Pulver (Verb. No. 2.01).

Durch Umkristallisieren der Mutterlaugen können noch weitere 58,2 g (23,7 %) der Titelverbindung isoliert werden.

### H. 2.2. Herstellung des Bisulfitaddukts des 2,4-Dichlorbutyraldehyds aus 3-Chlor-2-methoxytetrahydrofuran (II)

#### H. 2.2.1. unter Verwendung von Triethylamin als Katalysator

Zu 13,6 g (100 mMol) 3-Chlor-2-methoxytetrahydrofuran und 0,2 g (2 mMol) Triethylamin werden bei 80 - 85°C innerhalb von 2 Stunden 13,1 g (110 mMol) Thionylchlorid zugetropft. Der so erhältliche (1,2,4-Trichlorbut-1-yl)-methylether wird innerhalb von 30 Minuten bei 40 - 45°C 31,0 g (100 mMol) einer 33,5%igen wässrigen Natriumhydrogensulfitlösung und 6 ml Toluol gegeben, wobei der pH-Wert des Reaktionsgemischs mit 30%iger NaOH-Lösung bei pH 3,0 bis 3,5 gehalten wird. Anschliessend wird das Reaktionsgemisch auf 70 - 75°C erwärmt, die Toluolphase abgetrennt und die wässrige Phase auf Raumtemperatur abgekühlt, wobei das Produkt ausfällt.

Man isoliert 68,3 g (94,8 %) der Titelverbindung der Formel

$$Cl-CH_2-CH_2-CHCl-\overset{\overset{\displaystyle OH}{|}}{C}H-SO_3^{\ominus} \qquad Na^{\oplus}$$

als 34%ige wässrige Suspension (Verb. No. 2.01).

H. 2.2.2. Unter Verwendung von Lutidin als Katalysator

Analog zu Beispiel H. 2.2.1. erhält man aus 13,6 g (100 mMol) 3-Chlor-2-methoxytetrahydrofuran, 0,1 g (2 mMol) Lutidin und 13,1 g (110 mMol Thionylchlorid, 31,0 g (100 mMol) 33,5%iger wässriger Natriumbisulfitlösung und 6 ml Toluol 66,1 g (91,7 %) der Titelverbindung der Formel

$$Cl-CH_2-CH_2-CHCl-\overset{OH}{\overset{|}{C}}H-SO_3^{\ominus} \qquad Na^{\oplus}$$

in Form einer 34%igen wässrigen Bisulfitadduktsuspension (Verb. No. 2.01).

$^1$H-NMR (360 MHz, $D_2O$): $\delta$ = 2,1 - 2,45 und 2,5 - 2,65 (2m, 2H-C(3)); 3,7 - 3,95 (m, 2H-C(4)); 4,55 - 4,9 (3 komplexe Signale, H-C(1) und H-C(2)).

Analog zu den Herstellungsbeispielen H 2 erhält man die Verbindungen der Tabelle II.

Tabelle II

Verbindungen der Formel

$$X'-CH_2-CH_2-\overset{X''}{\overset{|}{C}}H-\overset{OH}{\overset{|}{C}}H-SO_3^{\ominus} \qquad Na^{\oplus}$$

| Verb. Nr. | X' | X'' | Ausbeute [%] |
|---|---|---|---|
| 2.01 | Cl | Cl | 95 |
| 2.02 | Br | Cl | |
| 2.03 | Br | Br | |
| 2.04 | Cl | Br | 90 1) |

1) aus 3-Brom-2-methoxytetrahydrofuran mit $SOCl_2$ als Halogenierungsmittel und Pyridin als Katalysator.

H. 3. Herstellung der Cyclopropancarbaldehyde I

H. 3.1. 1-Methylthiocyclopropancarbaldehyd

Zu 98,5 g (225 mMol) einer 16%igen wässrigen Natriummethylthiolatlösung und 60,0 g (450 mMol) einer 30%igen wässrigen Natriumhydroxidlösung werden bei 0 - 5°C 42,8 g (175 mMol) 2,4-Dichlorbutyraldehyd-Bisulfitaddukt (suspendiert in 200 ml Wasser) innerhalb von einer Stunde getropft. Es wird noch 45 Minuten nachgerührt und dann mit 100 ml Dichlormethan versetzt und von der wässrigen Phase abgetrennt. Die organische Phase wird am Rotationsverdampfer im Vakuum (50°C/500 mbar) eingedampft.

Man isoliert 19,4 g (95,6 %) der Titelverbindung der Formel

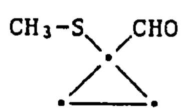

(Verbindung Nr. 4.01).

In analoger Weise isoliert man die Verbindungen der Tabelle III

Tabelle III

| Beispiel | Edukt | $R^1S^{\ominus}Na^{\oplus}$ | Produkt | Ausbeute |
|---|---|---|---|---|
| H.3.1.2. | OH<br>$BrCH_2-CH_2CHClCHSO_3Na$ | $CH_3SNa$ | 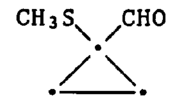 | |
| H.3.1.3. | Br OH<br>$ClCH_2CH_2CH-CHSO_3Na$ | $CH_3SNa$ | 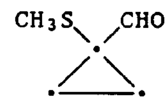 | 90 % |
| H.3.1.4. | Br OH<br>$BrCH_2CH_2CH-CHSO_3Na$ | $CH_3SNa$ | 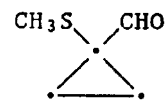 | |
| H.3.1.5. | Cl OH<br>$ClCH_2CH_2CH-CHSO_3Na$ | $CH_3(CH_2)_3SNa$ | 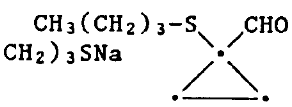 | 69 % |
| H.3.1.6. | Cl OH<br>$ClCH_2CH_2CH-CHSO_3Na$ | $C_6H_5-CH_2SNa$ | 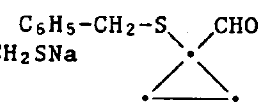 | 58 % |

H. 3.2. 1-Methylthiocyclopropancarbaldehyd aus 3-Chlor-2-methoxytetrahydrofuran ohne Isolierung der Zwischenstufen

273,0 g (2,0 Mol) 3-Chlor-2-methoxytetrahydrofuran, 3,16 g (40 mMol) Pyridin, 261,8 g (2,2 Mol) Thionylchlorid, 620 g (2,0 Mol) 33,5%ige wässrige Natriumhydrogensulfitlösung und 100 ml Toluol werden analog zu Beispiel H. 2.2. zu einer wässrigen Suspension des 2,4-Dichlorbutyraldehyd-Bisulfitaddukts umgesetzt. Die so erhältliche wässrige Suspension wird mit 210 ml Wasser verdünnt und auf 0°C gekühlt. Danach werden 876,3 g (2,0 Mol) einer 16%igen Natriummethylthiolatlösung und 533,0 g (4,0 Mol) einer 30%igen wässrigen Natriumhydroxidlösung innerhalb von 3 Stunden bei 0 - 5°C zugetropft. Es wird noch 45 Minuten bei 0 - 5°C nachgerührt, auf Raumtemperatur erwärmt, mit Toluol oder Dichlormethan extrahiert, von der wässrigen Phase abgetrennt und die organische Phase am Rotationsverdampfer im Vakuum eingeengt.

Man isoliert 185,7 g (80 %) der Titelverbindung der Formel

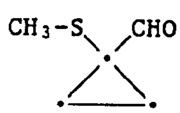

(Verb. Nr. 4.01).

Analog zu den vorstehenden Herstellungsbeispielen können die Verbindungen der Tabelle IV hergestellt werden:

Tabelle IV

Verbindungen der Formel

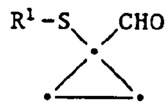

(I) ,

| Verb. Nr. | $R^1$ | Siedepunkt/Druck | Ausbeute |
|---|---|---|---|
| 4.01 | $CH_3$ | +91 bis +92°C/10000 Pa | 80 % |
| 4.02 | $CH_3(CH_2)_3-$ | | 61 % |
| 4.03 | $C_6H_5-CH_2-$ | +90 bis +91°C/1,3 Pa | 51 % |
| 4.04 | $C_2H_5$ | +57 bis +58°C/1800 Pa | |
| 4.05 | $C_3H_7$-n | +74 bis +76°C/2000 Pa | |
| 4.06 | $C_3H_7$-i | +67 bis +68°C/2000 Pa | |

## Patentansprüche

1. Verfahren zur Herstellung von Cyclopropancarbaldehyden der Formel

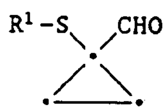

(I) ,

worin
$R^1$ $C_1$-$C_4$-Alkyl oder Benzyl bedeutet,
dadurch gekennzeichnet, dass man
a) ein Tetrahydrofuran der Formel

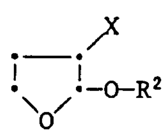

(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel zu einem 1,2,4-Trihalogenbutylether der Formel

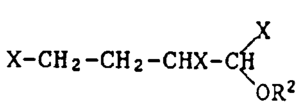

(III) ,

worin
X Halogen und

$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
umsetzt und
b) anschliessend die Verbindung der Formel III mit einer wässrigen Bisulfitlösung zu einem Bisulfitaddukt der Formel

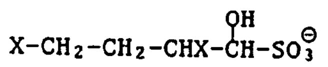 (IV),

worin
X Halogen und
Y ein Kationenäquivalent eines Alkali- oder Erdalkaliions bedeutet,
umsetzt und
c) anschliessend in Gegenwart einer Base mit einem Thiolat der Formel

$$R^1 - S^{\ominus} Z^{\oplus} \quad (V),$$

worin
$R^1$ $C_1$-$C_4$-Alkyl oder Benzyl und
Z ein Kationenäquivalent eines Alkali- oder Erdalkaliions bedeutet,
zu einem Cyclopropancarbaldehyd der Formel I cyclisiert.

2. Verfahren zur Herstellung von Cyclopropancarbaldehyden der Formel

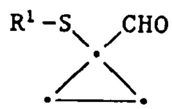 (I) ,

worin
$R^1$ $C_1$-$C_4$-Alkyl oder Benzyl bedeutet,
dadurch gekennzeichnet, dass man
a) ein Tetrahydrofuran der Formel

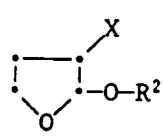 (II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit Thionylchlorid zu einem 1,4-Dichlor-2-halogenbutylether der Formel

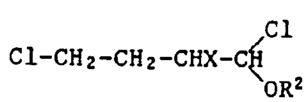 (IIIa) ,

worin
X Halogen und
$R^2$ gegebenenfalls halogensubstituiertes $C_1$-$C_{10}$-Alkyl, gegebenenfalls durch Halogen, $C_1$-$C_4$-Alkyl

oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
umsetzt und
b) anschliessend die Verbindung der Formel IIIa mit einer wässrigen Bisulfitlösung zu einem Bisulfitaddukt der Formel

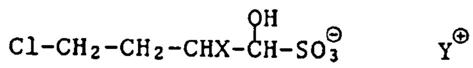
(IVa),

worin
X Halogen und
Y ein Kationenäquivalent eines Alkali- oder Erdalkaliions bedeutet,
umsetzt und
c) anschliessend in Gegenwart einer Base mit einem Thiolat der Formel

$$R^1 - S^{\ominus} Z^{\oplus} \quad (V),$$

worin
$R^1$ $C_1$-$C_4$-Alkyl oder Benzyl und
Z ein Kationenäquivalent eines Alkali- oder Erdalkaliions bedeutet,
zu einem Cyclopropancarbaldehyd der Formel I cyclisiert.

3. Verfahren zur Herstellung von Cyclopropancarbaldehyden der Formel

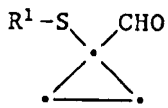
(I),

worin
$R^1$ $C_1$-$C_4$-Alkyl oder Benzyl bedeutet,
dadurch gekennzeichnet, dass man
b) einen 1,2,4-Trihalogenbutylether der Formel

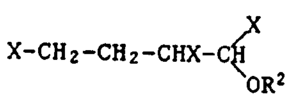
(III),

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einer wässrigen Bisulfitlösung zu einem Bisulfitaddukt der Formel

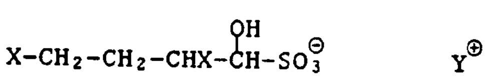
(IV),

worin
X Halogen und
Y ein Kationenäquivalent eines Alkali- oder Erdalkaliions bedeutet,
umsetzt
und
c) danach in Gegenwart einer Base mit einem Thiolat der Formel

$R^1 - S^{\ominus} Z^{\oplus}$ (V) ,

worin
$R^1$ $C_1$-$C_4$-Alkyl oder Benzyl und
Z ein Kationenäquivalent eines Alkali- oder Erdalkaliions bedeutet, zu einem Cyclopropancarbaldehyd der Formel I cyclisiert.

**4.** Verfahren nach Anspruch 3, dadurch gekennzeichnet, dass man
b) ein 1,4-Dichlor-2-halogenbutylether der Formel

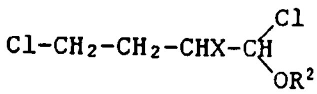
(IIIa) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einer wässrigen Bisulfitlösung zu einem Bisulfitaddukt der Formel

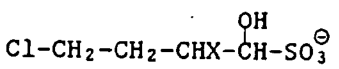
$Y^{\oplus}$ (IVa),

worin
X Halogen und
Y ein Kationenäquivalent eines Alkali- oder Erdalkaliions bedeutet,
umsetzt
und
c) danach in Gegenwart einer Base mit einem Thiolat der Formel

$R^1 - S^{\ominus} Z^{\oplus}$ (V) ,

worin
$R^1$ $C_1$-$C_4$-Alkyl oder Benzyl und
Z ein Kationenäquivalent eines Alkali- oder Erdalkaliions bedeutet, zu einem Cyclopropancarbaldehyd der Formel I cyclisiert.

**5.** Verfahren zur Herstellung eines Bisulfitaddukts der Formel

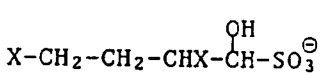
$Y^{\oplus}$ (IV),

worin
X Halogen und
Y ein Kationenäquivalent eines Alkali- oder Erdalkaliions bedeutet,
dadurch gekennzeichnet, dass man
a) ein Tetrahydrofuran der Formel

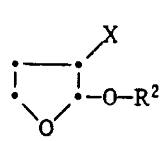
(II) ,

worin

X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel zu einem 1,2,4-Trihalogenbutylether der Formel

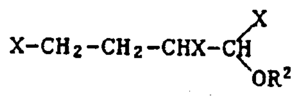

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet, umsetzt und
b) anschliessend die Verbindung der Formel III mit einer wässrigen Bisulfitlösung zu dem Bisulfitaddukt der Formel IV umsetzt.

6. Verfahren zur Herstellung von 1,2,4-Trihalogenbutylethern der Formel

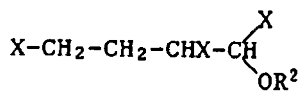

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
dadurch gekennzeichnet, dass man
a) ein Tetrahydrofuran der Formel

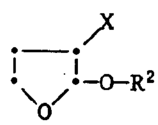

(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einem Halogenierungsmittel umsetzt.

7. Verfahren zur Herstellung eines Bisulfitaddukts der Formel

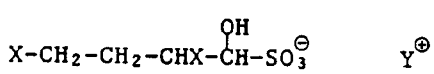

(IV),

worin
X Halogen und
Y ein Kationenäquivalent eines Alkali- oder Erdalkaliions bedeutet,
dadurch gekennzeichnet, dass man

b) einen 1,2,4-Trihalogenbutylether der Formel

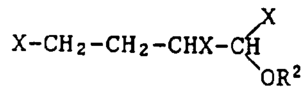

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit einer wässrigen Bisulfitlösung umsetzt.

**8.** Verfahren zur Herstellung von Cyclopropancarbaldehyden der Formel

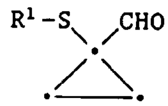

(I) ,

worin
$R^1$ $C_1$-$C_4$-Alkyl oder Benzyl bedeutet,
dadurch gekennzeichnet, dass man
c) ein Bisulfitaddukt der Formel

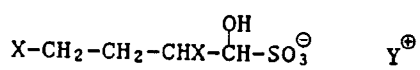

(IV),

worin
X Halogen und
Y ein Kationenäquivalent eines Alkali- oder Erdalkaliions bedeutet,
mit einem Thiolat der Formel

$$R^1 - S^\ominus\ Z^\oplus \quad (V),$$

worin
$R^1$ $C_1$-$C_4$-Alkyl oder Benzyl und
Z ein Kationenäquivalent eines Alkali- oder Erdalkaliions bedeutet, in Gegenwart einer Base zu einem Cyclopropancarbaldehyd der Formel I cyclisiert.

**9.** 1,2,4-Trihalogenbutlyether der Formel

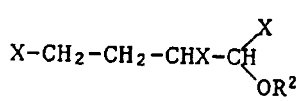

(III) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet.

**10.** 1,4-Dichlor-2-halogenbutylether der Formel

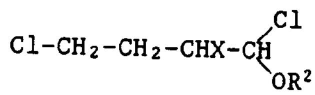

(IIIa) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet.

**11.** Bisulfitaddukte der Formel

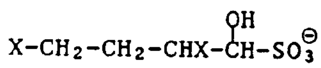

$Y^{\oplus}$ (IV),

worin
X Halogen und
Y ein Kationenäquivalent eines Alkali- oder Erdalkaliions bedeutet.

**12.** Bisulfitaddukte der Formel

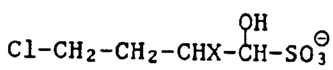

$Y^{\oplus}$ (IVa),

worin
X Halogen und
Y ein Kationenäquivalent eines Alkali- oder Erdalkaliions bedeutet.

**13.** Verbindungen der Formel II

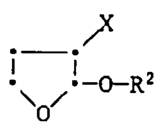

(II) ,

worin
X Halogen und
$R^2$ gegebenenfalls halogensubstituiertes $C_1$-$C_{10}$-Alkyl, gegebenenfalls durch Halogen, $C_1$-$C_4$-Alkyl oder $C_1$-$C_4$-Alkoxy substituiertes $C_3$-$C_6$-Cycloalkyl, oder gegebenenfalls durch Halogen, $C_1$-$C_4$-Alkyl, $C_1$-$C_4$-Alkoxy, Nitro, Cyano substituiertes Phenyl oder Benzyl bedeutet,
mit der Massgabe, dass
wenn X Chlor bedeutet $R^2$ nicht für unsubstituiertes $C_1$-$C_6$-Alkyl, Cyclohexyl, in 2-Stellung durch Chlor substituiertes $C_4$-$C_{10}$-Alkyl oder 4-Chlorbutyl steht oder wenn X Brom oder Iod bedeutet,
$R^2$ nicht für $C_1$-$C_4$-Alkyl steht.

## Claims

**1.** A process for the preparation of a cyclopropanecarbaldehyde of formula

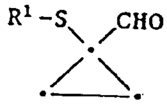

(I)

in which $R^1$ is $C_1$-$C_4$alkyl or benzyl, which comprises

a) reacting a tetrahydrofuran of formula

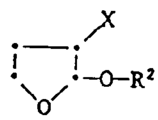

(II)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with a halogenating agent to form a 1,2,4-trihalobutyl ether of formula

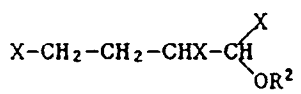

(III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, and

b) then reacting the compound of formula III with an aqueous bisulfite solution to form a bisulfite adduct of formula

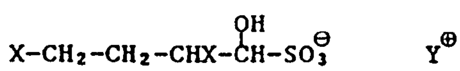

(IV)

in which X is halogen and Y is a cation equivalent of an alkali metal or alkaline earth metal ion, and

c) then cyclising the adduct, in the presence of a base, with a thiolate of formula

$R^1 - S^{\ominus} Z^{\oplus}$ (V)

in which $R^1$ is $C_1$-$C_4$alkyl or benzyl and Z is a cation equivalent of an alkali metal or alkaline earth metal ion, to form a cyclopropanecarbaldehyde of formula I.

**2.** A process for the preparation of a cyclopropanecarbaldehyde of formula

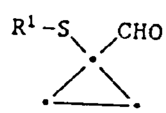

(I)

in which $R^1$ is $C_1$-$C_4$alkyl or benzyl, which comprises

a) reacting a tetrahydrofuran of formula

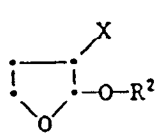

(II)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which

is unsubstituted or substituted by halogen, $C_1$-$C_4$alkyl, $C_1$-$C_4$alkoxy, nitro or by cyano, with thionyl chloride to form a 1,4-dichloro-2-halobutyl ether of formula

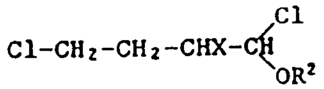

(IIIa)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, and
b) then reacting the compound of formula IIIa with an aqueous bisulfite solution to form a bisulfite adduct of formula

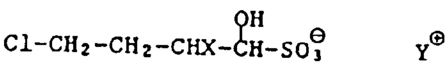

(IVa)

in which X is halogen and Y is a cation equivalent of an alkali metal or alkaline earth metal ion, and
c) then cyclising the adduct, in the presence of a base, with a thiolate of formula

$R^1 - S^{\ominus}\ Z^{\oplus}$ (V)

in which $R^1$ is $C_1$-$C_4$alkyl or benzyl and Z is a cation equivalent of an alkali metal or alkaline earth metal ion, to form a cyclopropanecarbaldehyde of formula I.

**3.** A process for the preparation of a cyclopropanecarbaldehyde of formula

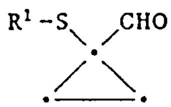

(I)

in which $R^1$ is $C_1$-$C_4$alkyl or benzyl, which comprises
b) reacting a 1,2,4-trihalobutyl ether of formula

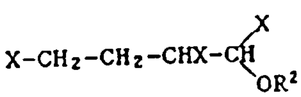

(III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with an aqueous bisulfite solution to form a bisulfite adduct of formula

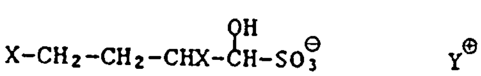

(IV)

in which X is halogen and Y is a cation equivalent of an alkali metal or alkaline earth metal ion and
c) subsequently cyclising the adduct, in the presence of a base, with a thiolate of formula

$R^1 - S^{\ominus}\ R^{\oplus}$ (V)

in which $R^1$ is $C_1$-$C_4$alkyl or benzyl and Z is a cation equivalent of an alkali metal or alkaline earth metal ion, to form a cyclopropanecarbaldehyde of formula I.

**4.** A process according to claim 3 which comprises
b) reacting a 1,4-dichloro-2-halobutyl ether of formula

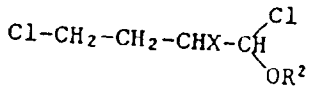 (IIIa)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with an aqueous bisulfite solution to form a bisulfite adduct of formula

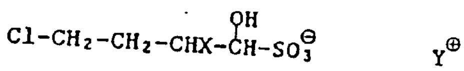 (IVa)

in which X is halogen and Y is a cation equivalent of an alkali metal or alkaline earth metal ion and
c) subsequently cyclising the adduct, in the presence of a base, with a thiolate of formula

$$R^1 - S^{\ominus} Z^{\oplus} \quad (V)$$

in which $R^1$ is $C_1$-$C_4$alkyl or benzyl and Z is a cation equivalent of an alkali metal or alkaline earth metal ion, to form a cyclopropanecarbaldehyde of formula I.

**5.** A process for the preparation of a bisulfite adduct of formula

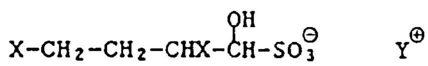 (IV)

in which X is halogen and Y is a cation equivalent of an alkali metal or alkaline earth metal ion, which comprises
a) reacting a tetrahydrofuran of formula

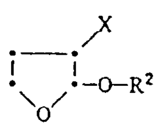 (II)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with a halogenating agent to form a 1,2,4-trihalobutyl ether of formula

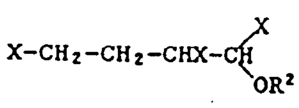 (III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, and
b) then reacting the compound of formula III with an aqueous bisulfite solution to form the bisulfite adduct of formula IV.

**6.** A process for the preparation of a 1,2,4-trihalobutyl ether of formula

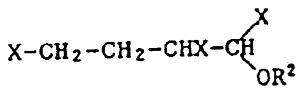

(III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, which comprises

a) reacting a tetrahydrofuran of formula

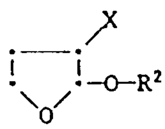

(II)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with a halogenating agent.

**7.** A process for the preparation of a bisulfite adduct of formula

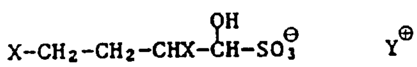

(IV)

in which X is halogen and Y is a cation equivalent of an alkali metal or alkaline earth metal ion, which comprises

b) reacting a 1,2,4-trihalobutyl ether of formula

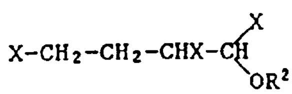

(III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with an aqueous bisulfite solution.

**8.** A process for the preparation of a cyclopropanecarbaldehyde of formula

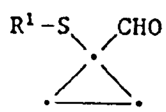

(I)

in which $R^1$ is $C_1$-$C_4$alkyl or benzyl, which comprises

c) cyclising a bisulfite adduct of formula

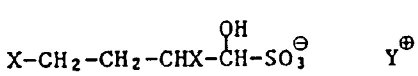

(IV)

in which X is halogen and Y is a cation equivalent of an alkali metal or alkaline earth metal ion, in the presence of a base, with a thiolate of formula

$$R^1 - S^{\ominus} Z^{\oplus} \quad (V)$$

in which $R^1$ is $C_1$-$C_4$alkyl or benzyl and Z is a cation equivalent of an alkali metal or alkaline earth metal ion, to form a cyclopropanecarbaldehyde of formula I.

**9.** A 1,2,4-trihalobutyl ether of formula

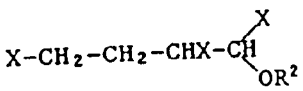

(III)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano.

**10.** A 1,4-dichloro-2-halobutyl ether of formula

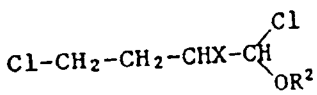

(IIIa)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano.

**11.** A bisulfite adduct of formula

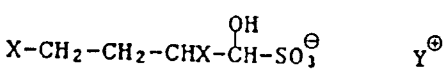

(IV)

in which X is halogen and Y is a cation equivalent of an alkali metal or alkaline earth metal ion.

**12.** A bisulfite adduct of formula

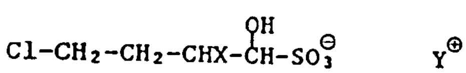

(IVa)

in which X is halogen and Y is a cation equivalent of an alkali metal or alkaline earth metal ion.

**13.** A compound of formula II

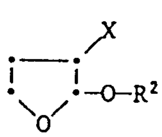

(II)

in which X is halogen and $R^2$ is unsubstituted or halosubstituted $C_1$-$C_{10}$alkyl, $C_3$-$C_6$cycloalkyl that is unsubstituted or substituted by halogen, $C_1$-$C_4$alkyl or by $C_1$-$C_4$alkoxy, or phenyl or benzyl each of which is unsubstituted or substituted by halogen, $C_1$-$C_4$alkyl, $C_1$-$C_4$alkoxy, nitro or by cyano, with the proviso that when X is chlorine $R^2$ is not unsubstituted $C_1$-$C_6$alkyl, cyclohexyl, $C_4$-$C_{10}$alkyl substituted in the 2-position by chlorine, or 4-chlorobutyl, and that when X is bromine or iodine $R^2$ is not $C_1$-$C_4$alkyl.

## Revendications

1. Procédé de préparation de cyclopropanecarbaldéhydes de formule

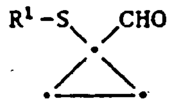

(I) ,

dans laquelle
$R^1$ représente un groupe alkyle en C1-C4 ou benzyle,
caractérisé en ce que
a) on convertit un tétrahydrofuranne de formule

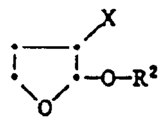

(II) ,

dans laquelle
X représente un halogène et
$R^2$ représente un groupe alkyle en C1-C10 éventuellement suhstitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
à l'aide d'un agent halogénant, en un éther 1,2,4-trihalogénobutylique de formule

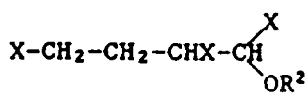

(III) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano et
b) on convertit ensuite le composé de formule III, à l'aide d'une solution aqueuse de bisulfite, en un adduct bisulfitique de formule

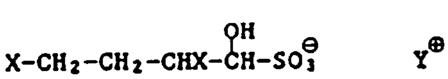

(IV),

dans laquelle
X représente un halogène et
Y représente un équivalent d'un cation alcalin ou alcalino-terreux et
c) on cyclise ensuite en présence d'une base, à l'aide d'un thiolate de formule

$$R^1 - S^{\ominus} Z^{\oplus} \quad (V),$$

dans laquelle
$R^1$ représente un groupe alkyle en C1-C4 ou benzyle et Z représente un équivalent d'un cation alcalin ou alcalino-terreux,
en un cyclopropane-carbaldéhyde de formule I.

2. Procédé de préparation des cyclopropane-carbaldéhydes de formule

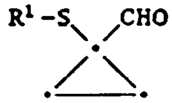

(I) ,

dans laquelle
$R^1$ représente un groupe alkyle en C1-C4 ou benzyle,
caractérisé en ce que

a) on convertit un tétrahydrofuranne de formule

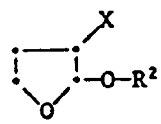

(II) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
à l'aide du chlorure de thionyle, en un éther 1,4-dichloro-2-halogénobutylique de formule

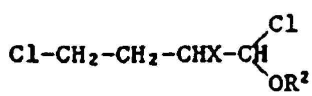

(IIIa) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano et

b) on convertit ensuite le composé de formule IIIa, à l'aide d'une solution aqueuse de bisulfite, en un adduct bisulfitique de formule

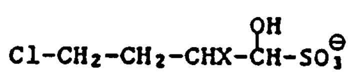

$Y^{\oplus}$ (IVa),

dans laquelle
X représente un halogène et
Y représente un équivalent d'un cation alcalin ou alcalino-terreux, et

c) on cyclise ensuite en présence d'une base, à l'aide d'un thiolate de formule

$R^1 - S^{\ominus} Z^{\oplus}$ (V) ,

dans laquelle
$R^1$ représente un groupe alkyle en C1-C4 ou benzyle et Z représente un équivalent d'un cation alcalin ou alcalino-terreux,
en un cyclopropane-carbaldéhyde de formule I.

3. Procédé de préparation des cyclopropane-carbaldéhydes de formule

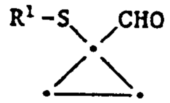

(I) ,

dans laquelle
$R^1$ représente un groupe alkyle en C1-C4 ou benzyle,
caractérisé en ce que

b) on convertit un éther 1,2,4-trihalogénobutylique de formule

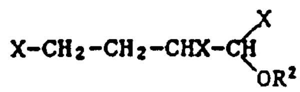

(III) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
à l'aide d'une solution aqueuse de bisulfite, en un adduct bisulfitique de formule

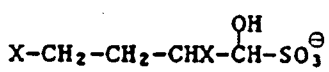

$Y^{\oplus}$ (IV),

dans laquelle
X représente un halogène et
Y représente un équivalent d'un cation alcalin ou alcalino-terreux et

c) on cyclise ensuite en présence d'une base, à l'aide d'un thiolate de formule

$$R^1 - S^{\ominus} Z^{\oplus} \quad (V) ,$$

dans laquelle
$R^1$ représente un groupe alkyle en C1-C4 ou benzyle et
Z représente un équivalent d'un cation alcalin ou alcalino-terreux,
en un cyclopropane-carbaldéhyde de formule I.

**4.** Procédé selon la revendication 3, caractérisé en ce que

b) on convertit un éther 1,4-dichloro-2-halogénobutylique de formule

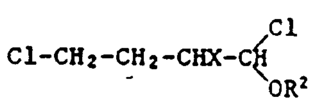

(IIIa) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
à l'aide d'une solution aqueuse de bisulfite, en un adduct bisulfitique de formule

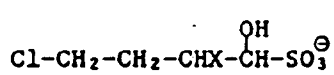

$Y^{\oplus}$ (IVa),

dans laquelle
X représente un halogène et
Y représente un équivalent d'un cation alcalin ou alcalino-terreux, et
c) on cyclise ensuite en présence d'une base, à l'aide d'un thiolate de formule

$$R^1 - S^{\ominus} Z^{\oplus} \quad (V),$$

dans laquelle
$R^1$ représente un groupe alkyle en C1-C4 ou benzyle et
Z représente un équivalent d'un cation alcalin ou alcalino-terreux,
en un cyclopropane-carbaldéhyde de formule I.

**5.** Procédé de préparation d'un adduct bisulfitique de formule

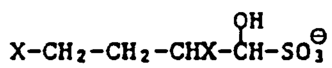

(IV),

dans laquelle
X représente un halogène et
Y représente un équivalent d'un cation alcalin ou alcalino-terreux,
caractérisé en ce que
a) on convertit un tétrahydrofuranne de formule

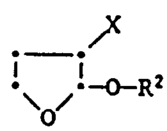

(II) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
à l'aide d'un agent halogénant, en un éther 1,2,4-trihalogénobutylique de formule

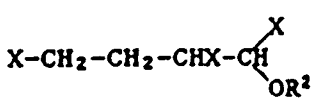

(III) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4,
b) on convertit ensuite le composé de formule III, à l'aide d'une solution aqueuse de bisulfite, en l'adduct bisulfitique de formule IV.

**6.** Procédé de préparation d'éthers 1,2,4-trihalogénobutyliques de formule

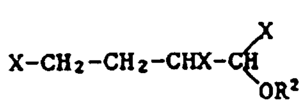

(III) ,

dans laquelle

X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano, caractérisé en ce que

a) on fait réagir un tétrahydrofuranne de formule

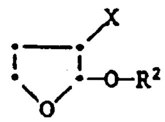

(II) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano, avec un agent halogénant.

**7.** Procédé de préparation d'un adduct bisulfitique de formule

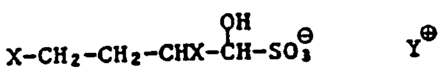

(IV),

dans laquelle
X représente un halogène et
Y représente un équivalent d'un cation alcalin ou alcalino-terreux,
caractérisé en ce que

b) on fait réagir un éther 1,2,4-trihalogénobutylique de formule

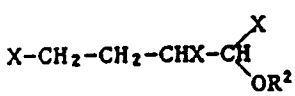

(III) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle, éventuellement substitué par des halogènes, des groupes alkyle en C1-C4, alcoxy en C1-C4, nitro, cyano,
avec une solution aqueuse de bisulfite.

**8.** Procédé de préparation de cyclopropanecarbaldéhydes de formule

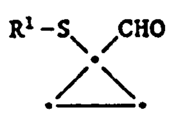

(I) ,

dans laquelle
$R^1$ représente un groupe alkyle en C1-C4 ou benzyle,
caractérisé en ce que

c) on cyclise un adduct bisulfitique de formule

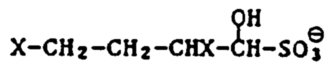 $Y^{\oplus}$ (IV),

dans laquelle
X représente un halogène et
Y représente un équivalent d'un cation alcalin ou alcalino-terreux,
à l'aide d'un thiolate de formule

$R^1 - S^{\ominus} Z^{\oplus}$ (V),

dans laquelle
$R^1$ représente un groupe alkyle en C1-C4 ou benzyle et
Z représente un équivalent d'un cation alcalin ou alcalino-terreux,
en présence d'une base, en un cyclopropane-carbaldéhyde de formule I.

**9.** Ethers 1,2,4-trihalogénobutyliques de formule

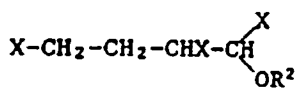 (III) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4 ou un groupe phényle ou benzyle éventuellement substitué par des halogènes, des groupes alkyle en C1-C4, alcoxy en C1-C4, nitro, cyano.

**10.** Ethers 1,4-dichloro-2-halogénobutyliques de formule

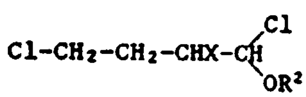 (IIIa) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano.

**11.** Adducts bisulfitiques de formule

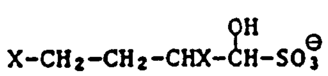 $Y^{\oplus}$ (IV),

dans laquelle
X représente un halogène et
Y représente un équivalent d'un cation alcalin ou alcalino-terreux.

**12.** Adducts bisulfitiques de formule

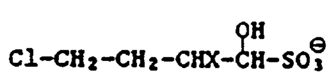 $Y^{\oplus}$ (IVa),

dans laquelle
X représente un halogène et
Y représente un équivalent d'un cation alcalin ou alcalino-terreux.

**13.** Composés de formule II

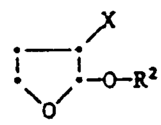

(II) ,

dans laquelle
X représente un halogène et
$R^2$ représente un groupe alkyle en C1-C10 éventuellement substitué par des halogènes, un groupe cycloalkyle en C3-C6 éventuellement substitué par des halogènes, des groupes alkyle en C1-C4 ou alcoxy en C1-C4, ou un groupe phényle ou benzyle éventuellement substitué par des halogènes, des groupes alkyle en C1-C4, alcoxy en C1-C4, nitro, cyano,
sous réserve que,
lorsque X représente le chlore, $R^2$ ne peut représenter un groupe alkyle en C1-C6 non substitué, un groupe cyclohexyle, un groupe alkyle en C4-C10 substitué en position 2 par le chlore ou un groupe 4-chlorobutyle, ou bien, lorsque X représente le brome ou l'iode, $R^2$ ne peut représenter un groupe alkyle en C1-C4.